# EUROPEAN PATENT APPLICATION

(11) **EP 3 644 550 A1**
(43) Date of publication of application: **29.04.2020**
(21) Application number: 18202501.5
(22) Date of filing: 25.10.2018
(51) Int. Cl.: H04L 9/32, G07C 9/00, H04W 12/08

(54) **UNLOCKING A MEDICAL DEVICE**

(71) Applicant: Siemens Healthcare GmbH, 91052 Erlangen (DE)
(72) Inventor: Bhandary Panambur, Adarsh, 91054 Erlangen (DE); Hager, Florian, 91058 Erlangen (DE); Kuth, Rainer, 91315 Höchstadt (DE)

(57) **Abstract**

At least one embodiment of the invention relates to a computer-implemented method for unlocking a medical device, comprising performing a check whether the medical device can access a distributed ledger, wherein the distributed ledger is stored in a plurality of nodes, and in the case of a positive check, unlocking the medical device. In particular, the step of performing a check is executed by a computation unit. In particular, the step of unlocking is executed by the computation unit. In particular, the computation unit is a computation unit of an unlocking system, of the medical device and/or of a control device. Further embodiments of the invention relate to an unblocking system, a medical device, a computer program product and a computer-readable storage medium.

## Description

In many technical areas it is known and common to log the access to technical devices as well as actions performed with the technical device, in order to be able to reconstruct modifications and/or manipulations of the technical devices. In particular, these logging techniques are used within security-critical technical areas, for example in aviation or in medical technology, and form part of a secure usage of these technical devices.

For example, modern implantable medical devices can be controlled and modified from the outside by means of a wireless communication, e.g. using a smartphone of a physician. However, access to or modifications of the implantable medical devices should be documented or logged in order to find the responsible persons in case of erroneous modifications.

It is known to use log files stored within the technical device or outside of the technical devices, for example as a text document. This has the disadvantage that log files can be altered, so that the ability to reconstruct modifications and/or manipulations of the technical devices can be reduced. In particular, these log files are susceptible for malicious entities and/or deliberate changes.

There are proposals to use blockchains or other types of distributed ledgers for logging access to technical devices and/or actions performed with technical devices. Here the blockchain or the distributed ledger is a tamper-proof and undisputable way for storing logs, by copies of the distributed ledger being stored in a plurality of nodes, and the synchronization of the plurality of nodes being ensured by a consensus algorithm. But using distributed ledgers for logging has the disadvantage that it cannot be ensured that certain data is actually entered into the distributed ledger (e.g. due to the consensus mechanism, or due to connectivity issues), decreasing the security of the technical device.

So it is the aim of the present invention to provide solutions for securely using technical devices.

The aim is solved by a method for unlocking a medical device, an unlocking system, a medical device, a computer program and a computer-readable medium according to the independent claims. Advantageous features are given in the dependent claims and the specification.

Although the invention is described with respect to medical devices, it has to be understood that the methods and systems disclosed in the claims and in the specifications can be used for all technical devices. In other words, the term "medical device" can be replaced with the term "technical device" within the specification and the claims wherever applicable, and the fact that a technical device is a medical device is not essential for the invention. Nevertheless, using the invention in context of medical devices is advantageous, because medical devices may harm a patient in the case of a malfunction, and are subject to strict security measures.

Examples for technical devices may be manufacturing machines, transport devices (e.g. cars, trains, planes, ships), building technology (e.g. light within buildings, heating and cooling, fire detectors), or other kinds devices used in all technical areas. In particular, technical devices may be devices may be technical devices in safety-critical areas.

In the following the solution according to the invention is described with respect to the claimed systems (in particular, with respect to the unlocking system, the medical device, the computer program and the computer-readable medium) as well as with respect to the claimed methods (in particular, with respect to the method for unlocking a medical device). Features, advantages or alternative embodiments herein can be assigned to the other corresponding claimed objects and vice versa. In other words, the systems can be improved with features described or claimed in the context of the corresponding method. In this case, the functional features of the methods are embodied by objective units of the systems.

According to a first aspect the invention relates to a computer-implemented method for unlocking a medical device, comprising performing a check whether the medical device can access a distributed ledger, wherein the distributed ledger is stored in a plurality of nodes, and in the case of a positive check, unlocking the medical device. In particular, the step of performing a check is executed by a computation unit. In particular, the step of unlocking is executed by the computation unit. In particular, the computation unit is a computation unit of an unlocking system, of the medical device and/or of a control device.

In general, a medical device is a technical apparatus for medical use. In particular, a medical device can execute or assist in executing medical procedures, in particular, the medical procedure being a diagnostic or a therapeutic procedure. In particular, a medical device is an active medical device, actively controlling a function of a body and/or being controllable from the outside. In particular, a medical device can also measure a condition of a human or animal body (e.g. the blood pressure, electrical signals from the heart and/or the brain), in particular by using a sensor. In particular, an implanted medical device is a medical device located within the human or animal body. In particular, the implanted medical device can be located within the human or animal body by an intervention. Examples for implanted medical devices are cardiac pacemakers, implantable cardioverter defibrillators, artificial hearts, implantable gastric stimulators, diaphragmatic/phrenic nerve stimulators or cochlear implants. In particular, a medical device can also be interpreted as a combination of an implanted medical device and another technical device (e.g. a smartphone, a computer) being connected with the medical device, in particular using a wireless connection. In particular, a medical device can comprise a controller, a sensor and an actor.

In particular, the medical device can comprise an acting medical device and a proxy medical device. In particular, the acting medical device directly interacts with the patients (i.e. by performing measurements and/or by acting on the patient), and the proxy device is configured for establishing a connection between the acting medical device and other entities, in particular between the acting medical device and the control device. In particular, the acting medical device can be implanted within the patient.

In general, the control device is a technical device for accessing, checking and/or manipulating the medical device. In particular, the control device can be used for assessing the status of the medical device, to set or changes parameters of the medical device, and/or to enable and disable certain functions of the medical device. In particular, the control device can be connected with the medical device, in particular using a wireless connection. If the medical device is a combination of an implanted medical device and another technical device, in particular the control device can interact with the implanted medical device using the another technical device as proxy.

In particular, by unlocking the medical device a certain function of the medical device is unlocked. In particular, by unlocking the medical device or the certain function of the medical device, the medical device or the certain function of the medical device can be activated. The certain function of the medical device can be a function associated with the medical functionality of the medical device, or a function associated with an administrative functionality of the medical device. In particular, by unlocking a medical device access to the medical device can be granted, for example, an interface for accessing the medical device can be unlocked, and/or it can be allowed to change certain parameters of the medical device.

In general, a distributed ledger is a certain type of a decentralized database. In particular, the distributed ledger is distributed in the sense that there are several copies of (at least parts of) the distributed ledger in different memory units, wherein the memory units are spatially or geographically distributed. The distributed ledger comprises multiple records, wherein the multiple records can be identified with database entries. In particular, the multiple records are organized as data blocks. In particular, the records are created by different entities, in particular different nodes of a network, and stored with the different entities, in particular within the different nodes of the records. In other words, the construction and maintenance of the records is typically not performed by a central authority, but independently by nodes of the network. In typical cases, each node of the network maintains a local copy of the distributed ledger.

In general, updating the distributed ledger is typically based on a consensus mechanism, wherein a consensus mechanism ensures that the different copies of the distributed ledger match, also in the cases of a delayed communication between the entities storing the copies of the distributed ledger.

In particular, storing data in a distributed ledger can comprise appending a further data block to the distributed ledger, wherein the further data block comprises the data to be stored. Appending a further data block to the distributed ledger can comprise executing the consensus mechanism, wherein executing the consensus mechanism can comprise a proof of work, a proof of storage, a proof of stake, and/or a proof of elapsed time. A proof of work can be a compute-bound proof of work, a network-bound proof of work and/or a memory-bound proof of work.

In general, data blocks are the elementary data records of the distributed ledger. In particular, a data block is an immutable data structure, which means that a data block cannot be changed or modified. This implies that changes to the distributed ledger can only be performed by adding or removing data blocks. In particular, the immutability of a data block can be ensured by storing a hash value of the data block within the data block or outside the data block.

In particular, a data block can comprise an arbitrary number of transactions. In particular, a data block can comprise a fixed number of transactions, in particular, the fixed number can be one. Furthermore, a data block can comprise a hash of another data block.

By using a distributed ledger comprising data blocks different copies of the distributed ledger can be synchronized more efficiently. In particular, using immutable data blocks it can be made harder for someone to manipulate the distributed ledger.

The inventors recognized that by unlocking the medical device only in the case the medical device can access the distributed ledger it can be ensured that the device only works in a specific function if data can be retrieved from the distributed ledger or inserted into the distributed ledger, e.g. for granting access or for logging access to the medical device.

According to a further possible aspect of the invention the method comprises, in the case of a negative check, locking the medical device. In particular, by locking the medical device a certain function of the medical device is locked. In particular, by locking the medical device or the certain function of the medical device, the medical device or the certain function of the medical device can be deactivated. The certain function of the medical device can be a function associated with the medical functionality of the medical device, or a function associated with an administrative functionality of the medical device. In particular, by locking a medical device access to the medical device can be denied, for example, an interface for accessing the medical device can be locked, and/or it can be not allowed to change certain parameters of the medical device. In particular, locking the medical device can be executed with the computation unit. In particular, the computation unit is a computation unit of the unlocking system, of the medical device and/or of the control device.

According to a further aspect of the invention the method comprises determining a test transaction, sending the test transaction to at least one first node of the plurality of nodes, and receiving the distributed ledger from at least one second node of the plurality of nodes, wherein the check whether the medical device can access the distributed ledger is positive, if the distributed ledger comprises the test transaction. In particular, the steps of sending the test transaction and receiving the distributed ledger can be executed with an interface. In particular, the step of determining the test transaction can be executed with the computation unit. In particular, the interface is an interface of the unlocking system, of the medical device and/or of the control device. In particular, the computation unit is a computation unit of the unlocking system, of the medical device and/or the control device.

In particular, the first node of the plurality of nodes can be identical with the second node of the plurality of nodes. Alternatively, the first node of the plurality of nodes can be different from the second node of the plurality of nodes. In particular, the sending of the test transaction can be executed by sending the test transaction to the plurality of nodes. In particular, the receiving of the distributed ledger can be executed by receiving the distributed ledger from the plurality of nodes.

The inventors recognized that by being able to include a test transaction into the distributed ledger it can be guaranteed that the medical device can send and/or document data within the distributed ledger. In particular, by being able to document data within the distributed ledger, the medical device is able to log certain events within the distributed ledger, in particular the unlocking of the medical device, and/or the use of a certain function of the medical device, and/or data about the user accessing the medical device. In particular, the ability to document a test transaction can be seen as a strong hint that also an actual transaction can be documented in the distributed ledger.

According to a further aspect of the invention the distributed ledger comprises data blocks, and the distributed ledger comprises the test transaction if the distributed ledger comprises a test transaction data block, wherein the test transaction data block is a data block comprising the test transaction. In particular, the distributed ledger comprises the test transaction data block if the copy of the distributed ledger within a certain number of the plurality of nodes, in particular within a majority of the plurality of nodes or within all of the plurality of nodes, contains the test transaction data block. The inventors recognized that by the existence of a block documenting the test transaction the test transaction was effectively inserted into the distributed ledger, in particular into all copies of the distributed ledger. In particular, if the test transaction data block is contained in several copies of the distributed ledger, the probability for a malicious node being able to provide a manipulated copy of the distributed ledger can be reduced.

According to a further aspect of the invention every data block of the distributed ledger comprises a hash of a further data block of the distributed ledger.

In particular, a hash of a dataset is the result of the application of a hash function on the dataset or a combination of the dataset and other data. In particular, the hash function can take other arguments, for example a seed.

In general a hash function is a function that maps data of arbitrary size to data of a fixed size. In particular, the hash function is a cryptographic hash function. In particular, a cryptographic hash function is a deterministic function; in particular the output of the hash function does only depend on the input of the hash function. In particular, a cryptographic hash function can be calculated in a fast manner for all input values. In particular, a cryptographic hash function is only brute-force invertible, i.e. given the output of a cryptographic hash function it is only possible to calculate the corresponding input of the cryptographic hash function by calculating the cryptographic hash function for an large amount of input values (i.e. a brute-force attack). In other words, finding the input value corresponding to the output value of a cryptographic hash function is an intractable problem. In particular, finding a first input value and a second input value of a cryptographic hash function that lead to an identical output value is an intractable problem. In particular, a cryptographic hash function is scattering; i.e. even correlated inputs of the cryptographic hash function lead to uncorrelated outputs of the cryptographic hash function.

In particular, a hash function can calculate a Merkle root of the input data, in particular by computing hashes within a Merkle tree.

The inventors recognized that by each data block comprising a hash of a further data block the distributed ledger is more resilient against manipulations, because a manipulation in a first data blocks can be detected by inspecting a second data block, the second data block directly or indirectly containing a hash of the second data block (the second data block indirectly contains a hash of the first data block, if it contains the hash of a third data block, the third data block directly or indirectly containing a hash of the first data block).

According to a further aspect of the invention the method comprises receiving a block requirement, wherein the check is only positive of the test transaction data block fulfills the block requirement. In particular, the step of receiving the block requirement is executed by the interface. In particular, the interface is an interface of the unlocking system, of the medical device and/or of the control device. In particular, the block requirement can also be received by the block requirement being stored within the medical device. In particular, the block requirement can be a requirement directed to the content of a data block and/or to the relation of the data block to other data blocks of the distributed ledger. The inventor recognized that by imposing a requirement for the test transaction data block to be fulfilled, the security of the medical device can be increased, because manipulations of the medical device are harder to achieve.

According to a further aspect of the invention, every data block of the distributed ledger comprises a hash of a further data block of the distributed ledger, the block requirement comprises an integer number, the distributed ledger comprises a number of succeeding data blocks of the test transaction data block, wherein the test transaction data block fulfills the block requirement if the number of succeeding data blocks is equal to or larger than the integer number.

In particular, a first data block is the a succeeding data block of a second data block, if the first data block comprises a hash of the second data block, or if the first data block comprises a hash of a succeeding data block of the second data block. In other words, each first data block that directly or indirectly comprises a hash of the second data block is a succeeding data block of the second data block. In particular, if counting or determining the number of succeeding data blocks of a certain block, only succeeding data blocks forming a chain or the longest chain can be considered, in other words, forks are not considered in determining or counting the number of succeeding data blocks.

The inventors recognized that by the block condition being a number of succeeding data blocks of the transaction data the manipulation of distributed ledger or a malicious attack on the distributed ledger can be impeded, because the manipulation of a data block is harder for data blocks with higher number of succeeding data blocks than with lower number of succeeding data blocks.

According to a further aspect of the invention the test transaction comprises the transfer of an amount of cryptocurrency from a first account to a second account, and wherein the amount of cryptocurrency is zero and/or the first account is equal to the second account. In particular, the first account can also be considered as equal to the second account if the first and the second account are owned by the same entity. The inventors recognized that transactions in distributed ledgers often require that a transaction of cryptocurrency is contained, in order to be considered as valid. By transferring an amount of zero cryptocurrency, and/or by transferring the cryptocurrency from a first account to a second account the number of possible test transactions can be increased, because the amount of cryptocurrency in the first account does not decrease.

According to a further aspect of the invention the method comprises measuring a medical value with a sensor of the medical device, and in the case of the medical value fulfilling a condition, unlocking the medical device. In particular, the step of unlocking is executed with the computation unit. In particular, the computation unit is a computation unit of the unlocking system, of the medical device and/or the control device. In particular, the step unlocking the medical device can comprise all advantageous features of the step of unlocking the medical device in case of a positive check.

In particular, there can be a step of receiving the condition, in particular with the interface. Alternatively, the condition can also be stored within a memory unit, in particular within a memory unit of the medical device and/or of the control device. In particular, the condition can be an upper and/or a lower threshold for the medical value.

In particular, a medical value can be a medical value related to a patient or a medical value related to the medical device.

In particular, a medical value related to the patient is the result of a measurement performed on the patient. In particular, a medical value related to a patient relates to the condition of a human or animal body. In particular, the medical value related to the patient can be measured by a medical device, in particular by a sensor of the medical device. In particular, the medical value related to the patient can be given by one or several numbers corresponding to the condition of the human or animal body. Furthermore, the medical value related to the patient can also comprise measurements describing the temporal behavior of one or several numbers corresponding to the human or animal body. For example, the medical value related to the patient can be the cardiac frequency, the blood pressure, or the (time-dependence of the) voltages acquired in an electrocardiogram, an electroencephalogram, an electromyogram, or an electroneurogram or an electronystagmogram.

In general, a medical value related to the medical device is a property of the medical device. Preferably, for determining the medical value related to the medical device, a measurement of the medical device is performed by the medical device itself or from the outside, and/or a status of the medical device is determined. Examples for medical values related to the medical device are a serial number of the medical device, a model number of the medical device, a status code of the medical device, a parameter of the medical device, and/or the remaining battery capacity of the medical device. Furthermore, a medical value related to the medical device can also comprise historic values describing certain actions of the medical device (e.g. a log file, or flags indicating that certain actions have be performed by the medical device).

The inventors recognized that by unlocking the medical device in case a medical value fulfills the condition the medical device can be unlocked in cases of a medical emergency, even if the distributed ledger cannot be accessed by the medical device. This allows a modification of the medical device to end the medical emergency.

According to a further aspect of the invention, the distributed ledger is a blockchain, a blocktree and/or a tangle.

In particular, the distributed ledger is a tangle if the distributed ledger is a directed acyclic graph. In particular, the distributed ledger is a blocktree, if for each data block in the distributed ledger (except a single origin data block) there is exactly one parent (or predecessor) of said data block. In particular, the distributed ledger is a blockchain if for each data block in the distributed ledger (except a single origin data block) there is exactly one successor of said data block in the distributed ledger; and for each data block in the distributed ledger (except one block data block) there is exactly one parent of said data block in the distributed ledger.

The inventors recognized that by using a blockchain, a blocktree and/or a tangle existing distributed ledgers can easily be used. In particular, by using an existing distributed ledger with many users and/or nodes, the security and the resilience of the medical device increases, because distributed ledgers with a large number of participating users and/or nodes are harder to manipulate. For example, in the case of a compute-bound proof of work, a malicious entity must have more than 50% of the computation power of all nodes at his availability in order to manipulate the distributed ledgers.

According to a further possible aspect of the invention, determining a further data block of the distributed ledger, in particular determining the test transaction data block, comprises the execution of a consensus algorithm, in particular a proof of work, a proof of storage, a proof of elapsed time and/or a proof of stake.

In general, a proof of work is a measure required by an entity to perform an action in a system, in particular by a computer to perform an action within a network, to reduce the possible number of actions in a certain time interval. In general, proof of work can also be interpreted as an economic measure to deter denial of service attacks and other service abuses such as spam on a network by requiring some work from a system using a service. In general the action to be performed is an asymmetric action, meaning that the resources needed for performing the action are higher than resources needed for verifying that the action actually has been performed. In particular, a proof of work can be a challengeresponse proof of work or a solution-verification proof of work.

In particular, the action to be performed for a proof of work, in particular for a CPU bound proof of work, is calculating the inverse of a one-way function, in particularly by calculating the one-way function multiple times and checking whether the result of the one-way function has a desired property or equals a desired value. In general, a one-way function is a function that can computed by a polynomial time algorithm, but any polynomial time randomized algorithm that attempts to compute a pseudo-inverse for the one-time functions succeeds with negligible probability. Examples for one-way functions is the multiplication of prime numbers (the inverse is the factorization of integer numbers), the Rabin function, the discrete exponential (calculating an exponential modulo an integer, in particular a prime number, the inverse function is the discrete logarithm), cryptographic hash functions, integer multiplication of points on elliptic curves over finite fields, or one-way functions based random linear codes or based on the subset sum problem.

In general, a proof of elapsed time means that the creation of a new data block by a node is based on elapsed time in the node or in the calculation unit. In particular, a proof of elapsed time may be based on secure instruction execution. In particular, the secure instruction execution can be done my using a secure environment and utilizing trustworthy functions, in particular a secure hardware environment. Another name for the secure environment is "trusted execution environment"; an example for a trusted executed environment is the product feature "Software Guard Extensions". In particular, a trusted execution environment comprises central processing unit (an acronym is "CPU") instruction codes that allows user-level code to allocate private regions of memory (sometimes called enclaves) that are protected from processes running at higher privilege levels. In particular, the proof of elapsed time can be based on a timer or time counting function executed in such an enclave, so that it is not possible for any other process to manipulate the timer. In particular, the probability for creating a new block can be proportional to the elapsed time.

In general, proof of stake means that only one node or a small amount of nodes from a plurality of nodes can create a block that is accepted as valid from other nodes of the network. The selection of the one node or the small amount of nodes can depend on a random selection, an amount of digital items assigned to the nodes (in other words, an account balance assigned to or associated with a node), or an age of digital items assigned to the nodes. In particular, a digital item can be a token or a unit of a cryptocurrency, and an account can comprise several such tokens or units. Another word for the amount of such tokens or cryptocurrency units is size of stake.

The inventors recognized that by using a consensus algorithm in particular a proof of work, a proof of storage, a proof of elapsed time and/or a proof of stake, a manipulation of the distributed ledger is impeded for a malicious entity.

According to a second aspect the invention relates to an unlocking system, comprising - a computation unit, configured for performing a check whether the medical device can access a distributed ledger, wherein the distributed ledger is stored in a plurality of nodes,
furthermore configured for unlocking the medical device in the case of a positive check.

In particular, the unlocking system is configured to execute the previously described method and its aspects. The unlocking system is configured to execute the previously described method and its aspects by its computation unit being configured to execute the respective method steps.

The unlocking system can be realized as a data processing system or as a part of a data processing system. Such a data processing system can, for example, comprise a cloudcomputing system, a computer network, a computer, a tablet computer, a smartphone, a microprocessor or the like. The unlocking system can comprise hardware and/or software. The hardware can be, for example, a processor system, a memory system and combinations thereof. The hardware can be configurable by the software and/or be operable by the software.

In particular, the unlocking system can be identical with or a subpart of the medical device. In particular, the unlocking system can be identical with or a subpart of the control device. In particular, the unlocking system can be identical with or a subpart of a combination of the medical device and the control device.

According to a third aspect the invention relates to a medical device comprising an unlocking system according to the invention and its aspects. According to a further aspect of the invention, the medical device comprises an implantable and/or implanted medical device.

According to a fourth aspect the invention relates to a computer program comprising instructions which, when the program is executed by an unlocking system, cause the unlocking system to carry out the method according to the invention and its aspects.

According to a fifth aspect the invention relates to a computer-readable medium comprising instructions which, when executed by an unlocking system, cause the unlocking system to carry out the method according to the invention and its aspects.

The realization of the invention by a computer program product and/or a computer-readable medium has the advantage that already existing providing systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

The properties, features and advantages of this invention described above, as well as the manner they are achieved, become clearer and more understandable in the light of the following description and embodiments, which will be described in details in the context of the drawings. This following description does not limit the invention on the contained embodiments. Same components or parts can be labeled with the same reference signs in different figures. In general the figures are not for scale. In the following:
- Fig. 1: displays the interaction between a medical device, a control device and a distributed ledger,
- Fig. 2: displays the interaction between a medical device, a proxy device, a control device and a distributed ledger,
- Fig. 3: displays a flowchart of a first embodiment of the method for unlocking a medical device,
- Fig. 4: displays a flowchart of a second embodiment of the method for unlocking a medical device,
- Fig. 5: displays a first embodiment of a distributed ledger,
- Fig. 6: displays a second embodiment of a distributed ledger,
- Fig. 7: displays an unlocking system,
- Fig. 8: displays a medical device and a control device,
- Fig. 9: displays a medical device comprising an acting medical device and a proxy device, as well as a control device.

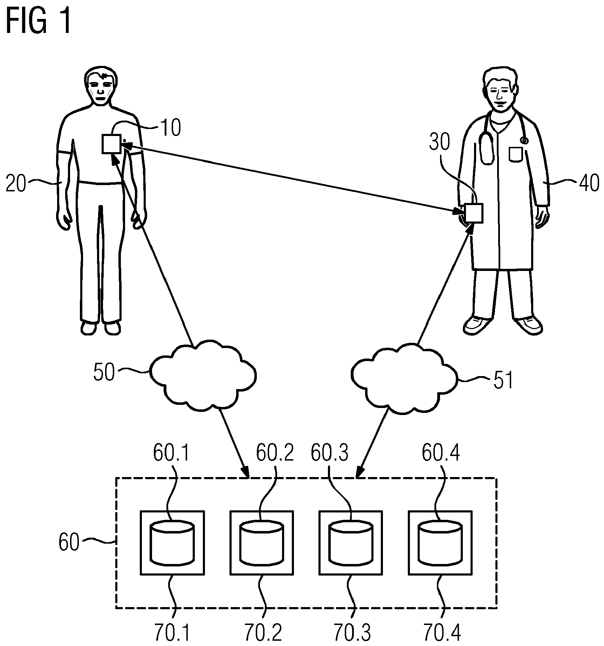

Fig. 1 displays the interaction between a medical device 10, a control device 30 and a distributed ledger 60.

In this embodiment, the medical device 10 is an implanted cardioverter defibrillator implanted into a patient 20. Alternatively, the medical device 10 can be another implanted or non-implanted medical device.

In particular, the medical device 10 being an implanted cardioverter defibrillator is an active medical device, measuring the electrocardiogram and/or the cardiac frequency using electrodes to discriminate between normal rhythm of the heart, supraventricular tachycardia, ventricular tachycardia, and ventricular fibrillation. Furthermore, the implanted cardioverter defibrillator can give electrical signals and/or shocks for cardioversion, defibrillation, and/or pacing of the heart of the patient 20.

The medical device 10 is controllable by a control device 30. In order to control the medical device 10, by means of the control device 30 parameters of the medical device 10 can be set, deleted and/or changed, the configuration of the medical device 10 can be changed and/or certain functions of the medical device 10 can be enabled or disabled. Usually the control device 30 is used by a physician 40.

In this embodiment, the control device 30 is a mobile device, e.g. a smartphone. Alternatively, the control device 30 can also be a stationary device. In order to control the medical device 10, the control device 30 is configured to communicate with the medical device 10, in particular by using a wireless communication, e.g. Bluetooth, WiFi, Medical Device Radiocommunications Service (an acronym is "MedRadio") or Medical Implant Communication Service / Medical Data Service (an acronym is "MICS/MEDS").

In order to document changes to the medical device 10 performed by the control device 30, the medical device 10 and/or the control device 30 can interact with a distributed ledger 60. In particular, the interaction with the distributed ledger 60 can take place via a first network 50 and a second network 51, wherein the first network 50 and the second network 51 can be different or identical networks. It is also possible that the medical device 10 interacts with the distributed ledger 60 by the control device 30 being a proxy. Alternatively, it is also possible that the control device 30 interacts with the distributed ledger 60 by the medical device 10 being a proxy.

In general, changes to the medical device 10 are documented in the distributed ledger 60 by including transactions 62.1, ..., 62.6, 65.1, ..., 65.3 and/or data blocks 61.1, ..., 61.6, 64.1, ..., 64.3 into the distributed ledger 60. Copies 60.1, ..., 60.4 of the distributed ledger 60 can be stored within the medical device 10, the control device 30 and within other nodes 70.1, ..., 70.4 contributing to the distributed ledger 60.

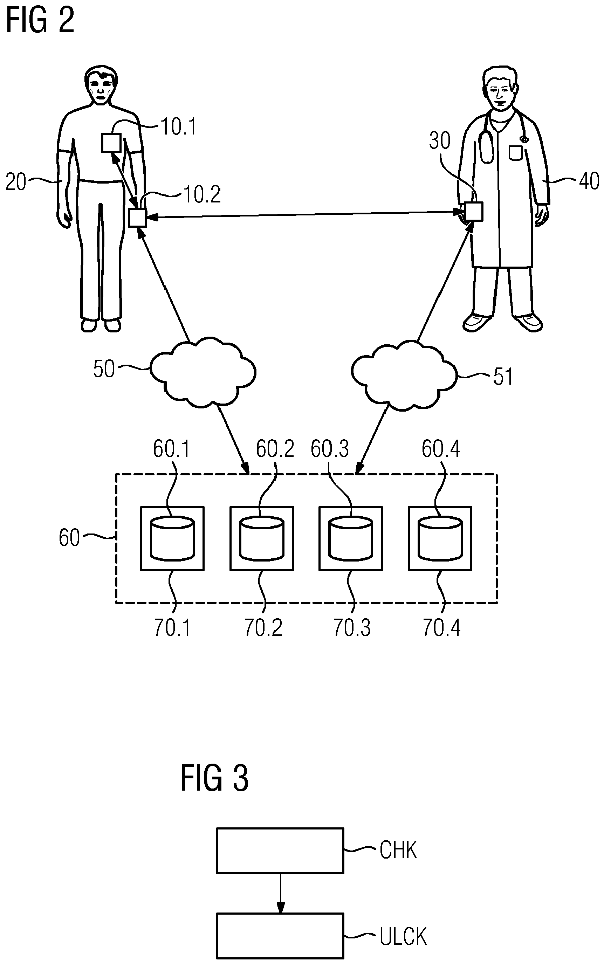

Fig. 2 displays a possible modification of the setup displayed in Fig. 1. In this embodiment, the medical device 10 comprises an acting medical device 10.1 and a proxy device 10.2. In particular, the acting medical device 10.1 and the proxy device 10.2 are in communication, preferably using a wireless connection. In particular, the acting medical device 10.1 cannot directly interact with the control device 30 or the distributed ledger 60, but an interaction can take place via the proxy device 10.2. In this embodiment, the acting medical device 10.1 is an implanted medical device, and the proxy device 10.2 is a mobile device owned and controlled by the patient 20, preferably a smartphone.

By using a separate acting medical device 10.1 and a proxy device 10.2, the security functionality can be included within the proxy device 10.2. In particular, since the proxy device 10.2 is located outside of the human body, certain restrictions (e.g. relating to the size or to the power consumption due to limited battery capacity) of implanted medical devices do not apply to the proxy device 10.2.

Fig. 3 displays a flowchart a first embodiment of the method for unlocking ULCK a medical device 10.

The first step of the displayed embodiment is performing a check CHK whether the medical device 10 can access a distributed ledger 60, wherein the distributed ledger 60 is stored in a plurality of nodes 70.1, ..., 70.4.

In this embodiment, the step of performing a check CHK is executed with the medical device 10, in particular with a computation unit 13 of the medical device 10. Alternatively, the step of performing a check CHK can also be executed with the control device 30, in particular with a computation unit 33 of the control device 30. In particular, in this case the control device 30 acts as a proxy for the medical device 10 to interact with the distributed ledger 60.

In this embodiment performing the check CHK comprises a check for read-access to the distributed ledger 60 and for write-access to the distributed ledger 60. In particular, a check for read-access to the distributed ledger 60 comprises checking whether a copy 60.1, ..., 60.4 of the distributed ledger 60 or of parts of the distributed ledger 60 can be accessed and/or downloaded. In particular, a check for write-access to the distributed ledger 60 comprises checking whether a transaction 62.1, ..., 62.6, 65.1, ..., 65.3 and/or a data block 61.1, ..., 61.6, 64.1, ..., 64.3 can be inserted into the distributed ledger 60, in particular into all or a certain subset of copies 60.1, ..., 60.4 of the distributed ledger 60.

The second step of the displayed embodiment is, in the case of a positive check, unlocking UNLCK the medical device 10. In this embodiment, in the case of a positive check the control device 30 is enabled to modify the medical device 10. In particular, certain access rights can be granted to the control device 30. Such modifications can comprise setting, deleting and/or changing of parameters of the medical device 10, and/or disabling and/or enabling certain functions of the medical device 10, and/or disabling and/or enabling the medical device 10 as such.

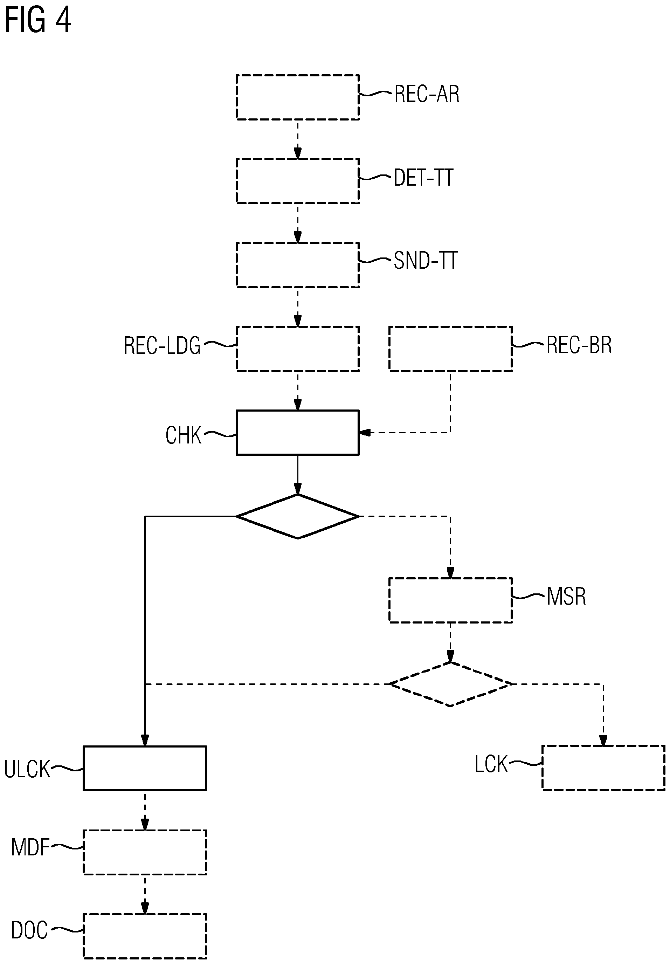

Fig. 4 displays a flowchart of a second embodiment of the method for unlocking ULCK a medical device 10.

The first optional step of the displayed embodiment is receiving REC-AR an access request for accessing the medical device 10. In particular, the access request is a dataset transmitted from the control device 30 to the medical device 10. In particular, the access request can comprise an identifier of the control device 30 or of the operator 40 of the control device 30, for example a serial number or a signature signed with a private key associated with the control device 30 or the operator 40,

The next optional step of the displayed embodiment is determining DET-TT a test transaction 65.1, ..., 65.3. In this embodiment, the step of determining DET-TT the test transaction 65.1, ..., 65.3 is executed with the computation unit 13 of the medical device 10. Alternatively, the step of determining DET-TT the test transaction 65.1, ..., 65.3 can also be executed by with the computation unit 33 of the control device 30.

In particular, the test transaction 65.1, ..., 65.3 can be an empty transaction or a null-transaction in the sense of the requirements of the distributed ledger 60. In particular, documenting the test transaction 65.1, ..., 65.3 within the distributed ledger 60 does not lead to further consequences. For example, if transactions 62.1, ..., 62.6, 65.1, ..., 65.3 in the distributed ledger 60 document transactions of certain tokens (e.g. cryptocurrency), the test transaction 65.1, ..., 65.3 can be a transaction of zero tokens or the transaction from a first account to a second account, wherein the first account and the second account are identical, or wherein the first account and the second account are controlled by the same entity. Alternatively, the test transaction 65.1, ..., 65.3 can also comprise data identifying itself as test transaction 65.1, ..., 65.3. For example, if the distributed ledger 60 logs access to medical devices 10 within data blocks 61.1, ..., 61.6, 64.1, ..., 64.3 and/or transactions 62.1, ..., 62.6, 65.1, ..., 65.3, the format of the data blocks 61.1, ..., 61.6, 64.1, ..., 64.3 and/or transactions 62.1, ..., 62.6, 65.1, ..., 65.3 can be chosen such that there is a flag indicating that a transaction 62.1, ..., 62.6, 65.1, ..., 65.3 is a test transaction 65.1, ..., 65.3.

The next optional step of the displayed embodiment is sending SND-TT the test transaction 65.1, ..., 65.3 to the plurality of nodes 70.1, ..., 70.4. In this embodiment, the test transaction 65.1, ..., 65.3 is sent as such to the nodes 70.1, ..., 70.4, and one of the nodes 70.1, ..., 70.4 determines a test transaction data block 64.1, ..., 64.3 comprising the test transaction 65.1, ..., 65.3. Alternatively, the medical device 10 determines a test transaction data block 64.1, ..., 64.3 comprising the test transaction 65.1, ..., 65.3, and sends the test transaction data block 64.1, ..., 64.3 to the nodes 70.1, ..., 70.4.

In both cases, determining a test transaction data block 64.1, ..., 64.3 comprising the test transaction 65.1, ..., 65.3 can comprise the execution of a consensus algorithm by the node 70.1, ..., 70.4 or by the medical device 10. In particular, the consensus algorithm can be a proof-of-work, a proof-of-stake and/or a proof-of-elapsed-time algorithm.

The next optional step of the displayed embodiment is receiving REC-60 the distributed ledger 60 from the plurality of nodes 70.1, ..., 70.4. In particular, the distributed ledger 60 can be received from one node 70.1, ..., 70.4 of the plurality of nodes 70.1, ..., 70.4. In particular, receiving REC-60 the distributed ledger 60 can also refer to receiving only certain data blocks 61.1, ..., 61.6, 64.1, ..., 64.3 of the distributed ledger 60.

In this embodiment, a request for downloading the distributed ledger 60 is sent to one node 70.1, ..., 70.4 of the plurality of nodes 70.1, ..., 70.4, for example by using a HTTP request (acronym for "Hypertext Transfer Protocol", a FTP request (acronym for "File Transfer Protocol"), or by using an API (acronym for "Application Programming Interface") of the one node 70.1, ..., 70.4 of the plurality of nodes 70.1, ..., 70.4. Alternatively, it is also possible that the distributed ledger 60 or data blocks 61.1, ..., 61.6, 64.1, ..., 64.3 of the distributed ledger 60 are automatically received in certain time intervals. In particular, a copy 60.1, ..., 60.4 of the distributed ledger 60 can be stored within a memory unit 14, 34, and new data blocks 61.1, ..., 61.6, 64.1, ..., 64.3 determined by nodes 70.1, ..., 70.4 are distributed to other nodes 70.1, ..., 70.4 of the system, including the medical device 10 or the control device 30.

The next step of the displayed embodiment is receiving REC-BR a block requirement, wherein the block requirement comprises an integer number. In this embodiment, the integer number specifies the number of second data blocks 61.1, ..., 61.6, 64.1, ..., 64.3 being successor of a first data block 61.1, ..., 61.6, 64.1, ..., 64.3 the distributed ledger 60 must comprise, in order to consider the first data block 61.1, ..., 61.6, 64.1, ..., 64.3 as validly and irrevocably inserted into the distributed ledger 60. For example, for the Bitcoin blockchain, this integer number would be six.

In this embodiment, the block requirement is stored within a memory unit 14, 34 of the medical device 10 or the control device 30. Alternatively, the block requirement can also be received in a timely relation to the execution of the other method steps.

In this embodiment, the number of second data blocks 61.1, ..., 61.6, 64.1, ..., 64.3 being successor of a first data block 61.1, ..., 61.6, 64.1, ..., 64.3 is determined based on the group of successor second data blocks 61.1, ..., 61.6, 64.1, ..., 64.3 forming the longest chain of successor second data blocks 61.1, ..., 61.6, 64.1, ..., 64.3.

The next step of the displayed embodiment is performing a check CHK whether the medical device 10 can access a distributed ledger 60, wherein the distributed ledger 60 is stored in a plurality of nodes 70.1, ..., 70.4. In this embodiment, the check is positive, if a test transaction data block 64.1, ..., 64.3 is documented or stored in the distributed ledger 60, and if test transaction data block 64.1, ..., 64.3 fulfills the block requirement. Alternatively, if there is no block requirement present, the check can be considered as positive if the test transaction data block 64.1, ..., 64.3 is documented or stored in the distributed ledger 60.

The test transaction data block 64.1, ..., 64.3 is a data block 61.1, ..., 61.6, 64.1, ..., 64.3 comprising the test transaction 65.1, ..., 65.3. In this embodiment, the test transaction data block 64.1, ..., 64.3 fulfills the block condition if the distributed ledger 60 comprises a certain number of data blocks 61.1, ..., 61.6, 64.1, ..., 64.3 being successors of the test transaction data block 64.1, ..., 64.3, and if said number is equal to or larger than the integer number contained in the block requirement.

In the case of a negative check, the next optional step in the displayed embodiment is measuring MSR a medical value with a sensor 15 of the medical device 10. In particular, the medical value is a medical value of the patient 20 the medical device 10 is implanted to. In this embodiment, the medical value comprises the time-dependency of voltage measured in an electrocardiogram.

If the medical value fulfills a predefined condition, for example the medical value is outside of a standard or normal interval, or the medical value indicates a severe problem with the patient 20, the next step of the displayed embodiment is unlocking ULCK the medical device 10. If the medical value does not fulfill the predefined condition, the next step of the displayed embodiment is locking LCK the medical device 10.

In this embodiment, the condition is fulfilled if the medical value (comprising a time-dependency of voltage measured in an electrocardiogram) indicates a supraventricular tachycardia, ventricular tachycardia, or a ventricular fibrillation. The condition is not fulfilled if the medical value indicates a normal cardiac activity.

Alternatively, the condition can also relate to the status of the medical device 10, in particular a status which can be assessed or measured MSR by the medical device 10. For example, the condition can be that the remaining battery lifetime of the medical device 10 is below a certain threshold, or that the medical device 10 has performed a certain activity (e.g. having applied a shock in the case of an implantable cardioverter defibrillator).

In the case of a positive check within the performing of the check CHK, or in the case the medical value fulfilling a certain condition, the next step of the displayed embodiment is unlocking ULCK the medical device 10. The unlocking ULCK can comprise all advantageous features and embodiments described with respect to Fig. 3 or the first embodiment of the method.

A further optional steps of the displayed embodiment is modifying MDF the medical device 10, in particular by setting, deleting and/or changing of parameters of the medical device 10, and/or disabling and/or enabling certain functions of the medical device 10, and/or disabling and/or enabling the medical device 10 as such.

A further optional step is documenting DOC the modifications within the distributed ledger 60, in particular by creating a log transaction 62.1, ..., 62.6 and/or a log transaction data block 61.1, ..., 61.6 comprising the log transaction 62.1, ..., 62.6, and by inserting the log transaction 62.1, ..., 62.6 and/or the log transaction data block 61.1, ..., 61.6 comprising the log transaction 62.1, ..., 62.6 into the distributed ledger 60.

In the case of a negative check within the performing of the check CHK, and in the case the medical value not fulfilling a certain condition (or, if the optional measuring is not performed at all), the next optional step of the displayed embodiment is locking LCK the medical device 10.

In this embodiment, within the step of locking LCK the control device 30 is disabled to modify the medical device 10. In particular, certain access rights can be denied to the control device 30. Such modifications can comprise setting, deleting and/or changing of parameters of the medical device 10, and/or disabling and/or enabling certain functions of the medical device 10, and/or disabling and/or enabling the medical device 10 as such.

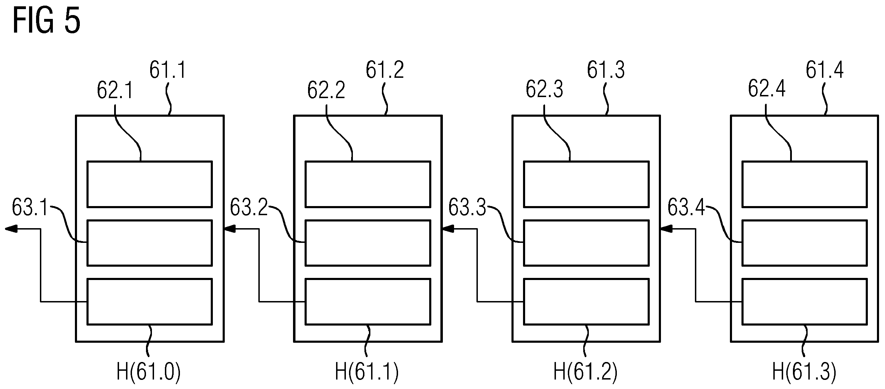

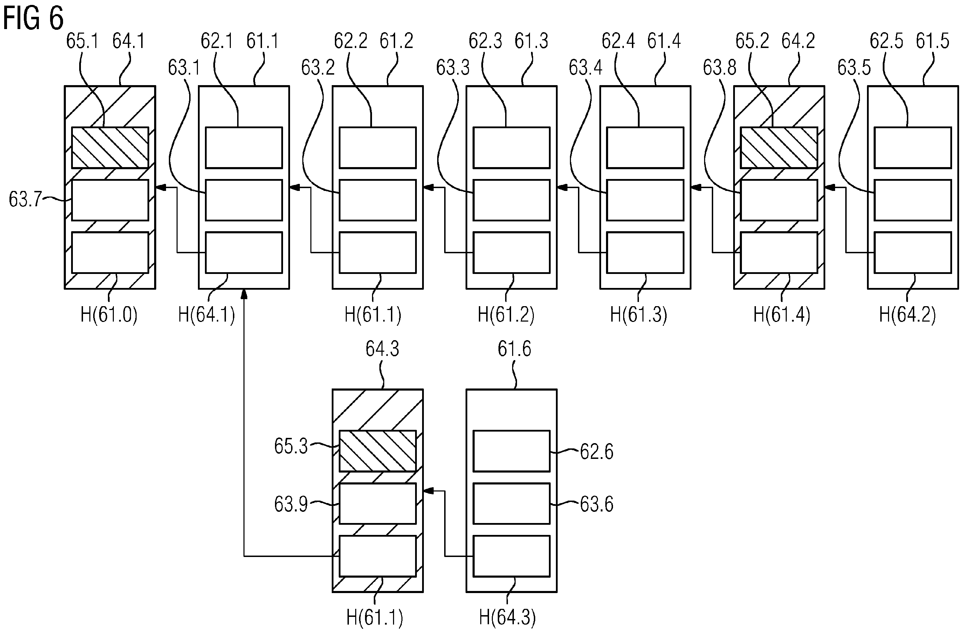

Fig. 5 and Fig. 6 display embodiments of a distributed ledger 60. In these embodiments, the distributed ledger 60 is a blockchain, which comprises data blocks 61.1, ..., 61.6, 64.1, ..., 64.3 forming a chain. Alternatively, other distributed ledgers 60 can be used, for example blocktrees (where the data blocks 61.1, ..., 61.6, 64.1, ..., 64.3 form a tree), or tangles (where the data blocks 61.1, ..., 61.6, 64.1, ..., 64.3 form a directed acyclic graph), or other forms of linked data blocks 61.1, ..., 61.6, 64.1, ..., 64.3. Within Fig. 5 only a part comprising four subsequent data blocks 61.1, ..., 61.6 is displayed, of course the distributed ledger 60 can comprise additional data blocks not displayed in Fig. 5. Within Fig. 6, the distributed ledger comprises additional data blocks 61.1, ..., 61.6, 64.1, ..., 64.3.

In these embodiments, the distributed ledger 60 comprises data blocks 61.1, ..., 61.6, 64.1, ..., 64.3, wherein each of the data blocks 61.1, ..., 61.6, 64.1, ..., 64.3 is linked to another data block 61.1, ..., 61.6, 64.1, ..., 64.3 (in the first embodiment displayed in Fig. 5, the data block 61.1 is linked to a data block which is not displayed, in the second embodiment displayed in Fig. 6, the data block 64.1 is linked to a data block which is not displayed). A link between two data blocks 61.1, ..., 61.6, 64.1, ..., 64.3 is a directional information, this means that for each link there is a well-defined start of the link and a well-defined end of the link. In particular, if a first data block 61.1, ..., 61.6, 64.1, ..., 64.3 is linked to a second data block 61.1, ..., 61.6, 64.1, ..., 64.3, this means that the first data block 61.1, ..., 61.6, 64.1, ..., 64.3 is the start of the link and the second data block 61.1, ..., 61.6, 64.1, ..., 64.3 is the end of the link. In other words, a first data block 61.1, ..., 61.6, 64.1, ..., 64.3 being linked to a second data block 61.1, ..., 61.6, 64.1, ..., 64.3 does not necessarily imply that the second data block 61.1, ..., 61.6, 64.1, ..., 64.3 is linked to the first data block 61.1, ..., 61.6, 64.1, ..., 64.3.

In these embodiments, each data block 61.1, ..., 61.6, 64.1, ..., 64.3 is linked to exactly one other data block 61.1, ..., 61.6, 64.1, ..., 64.3 (except one data block forming the origin of the distributed ledger 60 not displayed here), and each data block 61.1, ..., 61.3 (except the last data block 61.4 forming the end of the distributed ledger 60) is the end of exactly one link. Alternatively, a data block 61.1, ..., 61.6, 64.1, ..., 64.3 can be linked to several other data blocks 61.1, ..., 61.6, 64.1, ..., 64.3, and/or a data block 61.1, ..., 61.6, 64.1, ..., 64.3 can be the end of several links. In particular, each data block 61.1, ..., 61.6, 64.1, ..., 64.3 can be linked to a fixed number of other data blocks 61.1, ..., 61.6, 64.1, ..., 64.3. A data block 61.1, ..., 61.6, 64.1, ..., 64.3 can also be linked to itself.

In these embodiments, a first data block 61.1, ..., 61.6, 64.1, ..., 64.3 is linked to a second data block 61.1, ..., 61.6, 64.1, ..., 64.3 by the first data block 61.1, ..., 61.6, 64.1, ..., 64.3 comprising a hash H(61.0), ..., H(61.3), H(64.1), ..., H(64.3) of the second data block 61.1, ..., 61.6, 64.1, ..., 64.3. Alternatively, other link information can be included into the first data block 61.1, ..., 61.6, 64.1, ..., 64.3 to indicate that it is linked to the second data block 61.1, ..., 61.6, 64.1, ..., 64.3. In these embodiments, the hash H(61.0), ..., H(61.3), H(64.1), ..., H(64.3) is the result of the application of the SHA256 hash function on the second data block 61.1, ..., 61.6, 64.1, ..., 64.3. In particular, the SHA256 hash function is applied to a concatenation of the contents of the second data block 61.1, ..., 61.6, 64.1, ..., 64.3. In particular, for inserting a data block 61.1, ..., 61.6, 64.1, ..., 64.3 into the distributed ledger 60, a consensus algorithm (e.g. proof of work, proof of storage, proof of stake, proof of elapsed time) must be executed, wherein the consensus algorithm may be based on choosing the nonce 63.1, ..., 63.9 of the data block 61.1, ..., 61.6, 64.1, ..., 64.3 to be inserted. In particular, the nonce 63.1, ..., 63.9 is a data item (in particular a numbers) that can be chosen arbitrarily by the creator of the block. In these embodiments, the nonce 63.1, ..., 63.9 must be chosen by the creator of the data block 61.1, ..., 61.6, 64.1, ..., 64.3 such that the hash H(61.0), ..., H(61.3), H(64.1), ..., H(64.3) of said data block 61.1, ..., 61.6, 64.1, ..., 64.3 fulfills a certain condition. In these embodiments, the condition is that the hash H(61.0), ..., H(61.3), H(64.1), ..., H(64.3) of said data block 61.1, ..., 61.6, 64.1, ..., 64.3 is smaller than a given threshold.

In these embodiments, each data block 61.1, ..., 61.6, 64.1, ..., 64.3 comprises a transaction 62.1, ..., 62.6, 65.1, ..., 65.3. It is also possible that each data block 61.1, ..., 61.6, 64.1, ..., 64.3 comprises a plurality of transactions 62.1, ..., 62.6, 65.1, ..., 65.3. The transactions 62.1, ..., 62.6, 65.1, ..., 65.3 can be considered as being the actual data stored in the distributed ledger 60. In general, the distributed ledger 60 can comprise further data blocks 61.1, ..., 61.6, 64.1, ..., 64.3 not comprising data relevant to the methods and systems of this invention. In other word, a multi-purpose distributed ledger 60 can be used, for example the "Bitcoin" blockchain or the "Ethereum" blockchain. Furthermore, each of the data blocks 61.1, ..., 61.6, 64.1, ..., 64.3 of the distributed ledger 60 can comprise additional data, either data related to methods and systems of this invention, or data unrelated to methods and systems of this invention.

In these embodiments, a transaction 62.1, ..., 62.6, 65.1, ..., 65.3 comprises logs relating to medical devices 10, in particular relating to a plurality of medical devices. In particular, a transaction 62.1, ..., 62.6, 65.1, ..., 65.3 can comprise an identifier of a person accessing a medical device 10, an identifier of the medical device 10, a timestamp of the access to the medical device 10, the modifications performed with respect to the medical device 10, and/or other data related to the access to the medical device 10. In particular, a transaction 62.1, ..., 62.6, 65.1, ..., 65.3 can be a test transaction 65.1, ..., 65.3. In these embodiments, a test transaction 65.1, ..., 65.3 has a flag indicating that it is a test transaction 65.1, ..., 65.3 and not a standard transaction 62.1, ..., 62.6. Alternatively, a transaction 62.1, ..., 62.6, 65.1, ..., 65.3 being a test transaction 65.1, ..., 65.3 can be encoded within the identifier of the person accessing the medical device 10, the identifier of the medical device 10, the timestamp of the access to the medical device 10, and/or the modification performed with respect to the medical device 10. For example, a specific test identifier can be used, which can optionally be related to the identifier of the medical device 10 or the identifier of the person, which replaces the identifier of the medical device 10 or the identifier of the person. Alternatively, the data field corresponding to the modifications can comprise the entry "test" or "nothing".

In the second embodiment of the distributed ledger 60 displayed in Fig. 6, there are log data blocks 61.1, ..., 61.6 comprising log transactions 62.1, ..., 62.6, and test transaction data blocks 64.1, ..., 64.3 comprising test transactions 65.1, ..., 65.3. Furthermore, within the second embodiment the distributed ledger 60 forks into a main chain (comprising the log data blocks 61.1, ..., 61.5 and the test transaction data blocks 64.1, 64.2) and into a side chain (comprising the log data block 61.6 and the test transaction data block 64.3). In particular, the data validly documented within the distributed ledger 60 is considered to be the data documented within the data blocks 61.1, ..., 61.5, 64.1, 64.2 contained in the longest chain. In particular, if creating a new data block 61.1, ..., 61.6, 64.1, ..., 64.3, the creator of the data block 61.1, ..., 61.6, 64.1, ..., 64.3 would use the last block of the longest chain in the distributed ledger 60 as predecessor for the new data block 61.1, ..., 61.6, 64.1, ..., 64.3.

In particular, for determining number of succeeding data blocks 61.1, ..., 61.6, 64.1, ..., 64.3 of a certain data block 61.1, ..., 61.6, 64.1, ..., 64.3, preferably only data blocks 61.1, ..., 61.6, 64.1, ..., 64.3 contained in the longest chain of the distributed ledger 60 are considered. For example, for the test transaction data block 64.1 there are six succeeding data blocks 61.1, ..., 61.5, 64.2, for the test transaction data block 64.2 there is one succeeding data block 61.5, and for the test transaction data block 64.3 there is no succeeding data block (since the data block 61.6 is not contained in the longest chain).

By this way of counting the number of succeeding data blocks it can be ensured that creating several data blocks 61.1, ..., 61.6, 64.1, ..., 64.3 linked to a single test transaction data block 64.1, ..., 64.3 does not lead to the presumption that the test transaction data block 64.1, ..., 64.3 is validly documented within the distributed ledger 60.

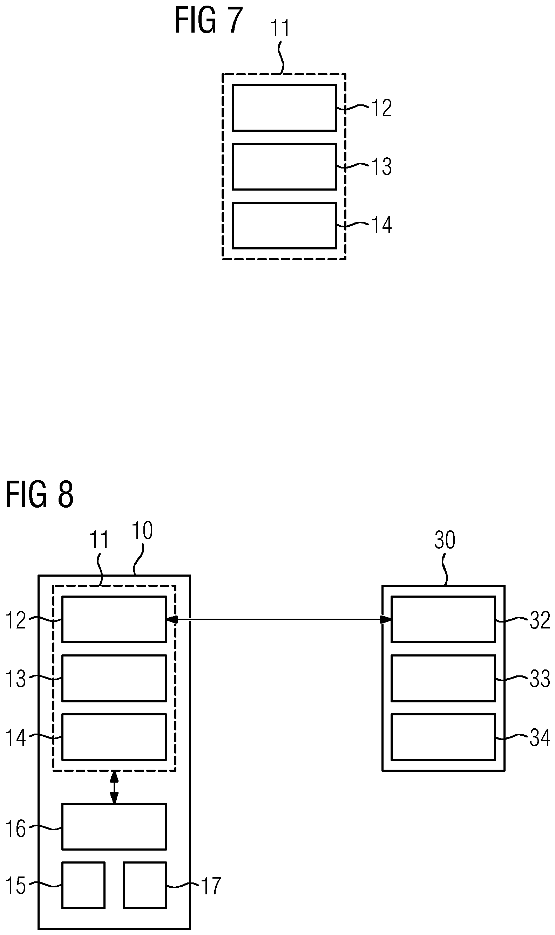

Fig. 7 displays an unlocking system 11. The unlocking system 11 can comprise a (personal) computer, a workstation or a virtual machine running on host hardware, a microcontroller, or an integrated circuit. Alternatively, the unlocking system 11 can be or comprise a real or a virtual group of computers (the technical term for a real group of computers is "cluster", the technical term for a virtual group of computers is "cloud").

In the displayed embodiment, the unlocking system 11 comprises an interface 12, a computation unit 13 and a memory unit 14.

An interface 12 can be embodied as a hardware interface or as a software interface (e.g. PCIBus, USB, Firewire, Bluethooth, WiFi). In particular, the interface 12 can be a hardware module configured for wireless communication. In particular, an interface 12 can be a combination of multiple distinct interfaces. In general, a computation unit 13 can comprise hardware elements and software elements, for example a microprocessor, a CPU (acronym for "central processing unit"), a GPU (acronym for "graphical processing unit"), a field programmable gate array (an acronym is "FPGA") or an ASIC (acronym for "application-specific integrated circuit"). In particular, a computation unit 13 can be a combination of multiple distinct computation units. A memory unit 14 can be e.g. non-permanent main memory (e.g. random access memory) or permanent mass storage (e.g. hard disk, USB stick, SD card, solid state disk).

The displayed unlocking system 11 can be contained in a medical device 10 and/or a control device 30.

Fig. 8 displays a medical device 10 and a control device 30. The medical device 10 comprises the unlocking system 11, a sensor 15, a controller 16 and an actor 17. The control device 30 comprises an interface 32, a computation unit 33, and a memory unit 34.

Alternatively, the unlocking system 11 can also be contained in the control device 30, or in the combination of the medical device 10 and the control device 30. In particular, the interface 32 of the control device 30 can comprise the interface 12 of the unlocking system 11, or the interface 12 of the unlocking system 11 can comprise the interface 32 of the control device 30. In particular, the computation unit 33 of the control device 30 can comprise the computation unit 13 of the unlocking system 11, or the computation unit 13 of the unlocking system 11 can comprise the computation unit 33 of the control device 30. In particular, the memory unit 34 of the control device 30 can comprise the memory unit 14 of the unlocking system 11, or the memory unit 14 of the unlocking system 11 can comprise the memory unit 34 of the control device 30.

The control device 30 can comprise a (personal) computer, a workstation or a virtual machine running on host hardware, a microcontroller, or an integrated circuit. Alternatively, the control device 30 can be or comprise a real or a virtual group of computers (the technical term for a real group of computers is "cluster", the technical term for a virtual group of computers is "cloud"). In particular, the medical device 10 and/or the control device 30 can be or comprise a mobile device, e.g. a smartphone or a tablet.

A sensor 15 detects events or changes in its environment and sends the information to other electronics, in particular to the computing unit 13 of the medical device 10. In particular, the sensor 15 is configured to measure a medical value, in particular a medical value related to the patient 20, or a medical value related to the medical device 10. For example, a sensor can be an electrode measuring the cardiac rate or an electrocardiogram.

In particular, the controller 16 of the medical device 10 can comprise hardware elements and software elements. In particular, the controller 16 can comprise a microcontroller, or an integrated circuit. Furthermore, the controller 16 can comprise a microprocessor, a CPU (acronym for "central processing unit"), a GPU (acronym for "graphical processing unit"), a field programmable gate array (an acronym is "FPGA") or an ASIC (acronym for "application-specific integrated circuit"). The medical device 10 can additionally comprise a (personal) computer, a workstation or a virtual machine running on host hardware, a microcontroller, or an integrated circuit. The control device 30 can be a microcontroller or an integrated circuit, a (personal) computer, a workstation or a virtual machine running on host hardware. In particular, the medical device 10 and the control device 30 can be or comprise a mobile device, e.g. a smartphone or a tablet. As an alternative, the control device 30 can be or comprise a real or a virtual group of computers (the technical term for a real group of computers is "cluster", the technical term for a virtual group of computers is "cloud").

In particular, the controller 16 can communicate with the unlocking system 11. In this embodiment, the controller 16 can communicate with entities outside of the medical device 10 (e.g. with the control device 30) via the unlocking system 11. It is also possible that the controller 16 can directly communicate with entities outside of the medical device 10.

In particular, an actor 17 can act on the patient 20. For example, an actor can be an electrode sending electrical signals, e.g. for regulating the cardiac rate, or to end a ventricular fibrillation. Alternatively, the actor can also be equipped to release drugs to the patient 20 (e.g. insulin or morphine).

The interface 32 can be embodied as a hardware interface or as a software interface (e.g. PCIBus, USB or Firewire). In particular, an interface 12, 32 can be a combination of multiple distinct interfaces. In general, the computation unit 33 can comprise hardware elements and software elements, for example a microprocessor, a CPU (acronym for "central processing unit"), a GPU (acronym for "graphical processing unit"), a field programmable gate array (an acronym is "FPGA") or an ASIC (acronym for "application-specific integrated circuit"). The computation unit 33 can be configured for multithreading, i.e. the computation unit 33 can host different calculation processes at the same time, executing the either in parallel or switching between active and passive calculation processes. In particular, the computation unit 33 can be a combination of multiple distinct computation units. A memory unit 34 can be e.g. non-permanent main memory (e.g. random access memory) or permanent mass storage (e.g. hard disk, USB stick, SD card, solid state disk).

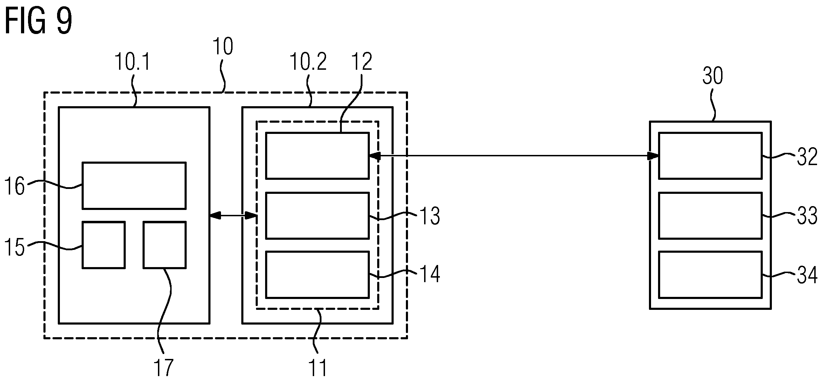

Fig. 9 displays a further embodiment of a medical device 10 and a control device 30. The medical device 10 comprises an acting medical device 10.1 and a proxy device 10.2. Here, the acting medical device 10.1 comprises the sensor 15, the controller 16 and the actor 17, and the proxy device 10.2 comprises the unlocking system 11.

In this embodiment, the acting medical device 10.1 as a cardiac pacemaker, and the proxy device 10.2 is the smartphone of the patient 20. The acting medical device 10.1 and the proxy device 10.2 are coupled. Preferably the acting medical device 10.1 is configured to be able to connect only to the proxy device 10.2, and not to another device. For example, the acting medical device 10.1 can store the a public key of a first asymmetric key pair and the private key of a second asymmetric key pair, and the proxy device 10.2 can store the corresponding private key of the first asymmetric key pair and the corresponding public key of the second asymmetric key pair, and interactions between the acting medical device 10.1 and the proxy device 10.2 are only allowed if they are signed by the respective private key.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

## Claims

1. A computer-implemented method for unlocking a medical device (10), comprising the following steps:
- performing a check (CHK) whether the medical device (10) can access a distributed ledger (60),
wherein the distributed ledger (60) is stored in a plurality of nodes (70.1, ..., 70.4), and
- in the case of a positive check, unlocking (ULCK) the medical device (10).

2. The method according to claim 1, furthermore comprising the following steps:
- determining (DET-TT) a test transaction (65.1, ..., 65.3),
- sending (SND-TT) the test transaction (65.1, ..., 65.3) to at least one first node (70.1, ..., 70.4) of the plurality of nodes (70.1, ..., 70.4),
- receiving (REC-LDG) the distributed ledger (60) from at least one second node (70.1, ..., 70.4) of the plurality of nodes (70.1, ..., 70.4);
wherein the check whether the medical device (10) can access the distributed ledger (60) is positive if the distributed ledger (60) comprises the test transaction (65.1, ..., 65.3).

3. The method according to claim 2, wherein the distributed ledger (60) comprises data blocks (61.1, ..., 61.6, 64.1, ..., 64.3),
wherein the distributed ledger (60) comprises the test transaction (65.1, ..., 65.3) if the distributed ledger (60) comprises a test transaction data block (64.1, ..., 64.3),
and wherein the test transaction data block (64.1, ..., 64.3) is a data block (61.1, ..., 61.6, 64.1, ..., 64.3) comprising the test transaction (65.1, ..., 65.3).

4. The method according to claim 3, wherein every data block (61.1, ..., 61.6, 64.1, ..., 64.3) of the distributed ledger (60) comprises a hash of a further data block (61.1, ..., 61.6, 64.1, ..., 64.3) of the distributed ledger (60).

5. The method according to claim 3 or 4, furthermore comprising:
- receiving (REC-BR) a block requirement, wherein the check is only positive if the test transaction data block (64.1, ..., 64.3) fulfills the block requirement.

6. The method according to claim 5, wherein every data block (61.1, ..., 61.6, 64.1, ..., 64.3) of the distributed ledger (60) comprises a hash of a further data block (61.1, ..., 61.6, 64.1, ..., 64.3) of the distributed ledger (60),
wherein the block requirement comprises an integer number, wherein the distributed ledger (60) comprises a number of succeeding data blocks (61.1, ..., 61.6, 64.1, ..., 64.3) of the test transaction data block (64.1, ..., 64.3),
and wherein the test transaction data block (64.1, ..., 64.3) fulfills the block requirement if the number of succeeding data blocks (61.1, ..., 61.6, 64.1, ..., 64.3) is equal to or larger than the integer number.

7. The method according to one of the claims 2 to 6, wherein the test transaction (65.1, ..., 65.3) comprises the transfer of an amount of cryptocurrency from a first account to a second account, and wherein the amount of cryptocurrency is zero and/or the first account is equal to the second account.

8. The method according to one of the preceding claims, furthermore comprising:
- measuring (MSR) a medical value with a sensor (15) of the medical device (10),
- in the case of the medical value fulfilling a condition, unlocking (ULCK) the medical device (10).

9. The method according to one of the preceding claims, wherein the distributed ledger (60) is a blockchain, a blocktree and/or a tangle.

10. An unlocking system (11), comprising
- a computation unit (13), configured for performing a check (CHK) whether the medical device (10) can access a distributed ledger (60),
wherein the distributed ledger (60) is stored in a plurality of nodes (70.1, ..., 70.4),
furthermore configured for unlocking (ULCK) the medical device (10) in the case of a positive check.

11. The unlocking system (11) according to claim 10, furthermore configured for executing the method according to one of the claims 2 to 9.

12. A medical device (10) comprising the unlocking system (11) according to claim 10 or 11.

13. The medical device (10) according to claim 12, the medical device (10) comprising an implantable and/or implanted medical device.

14. A computer program comprising instructions which, when the program is executed by an unlocking system (11), cause the unlocking system (11) to carry out the method of one of the claims 1 to 9.

15. A computer-readable medium comprising instructions which, when executed by an unlocking system (11), cause the unlocking system (11) to carry out the method of one of the claims 1 to 9.
